# EUROPÄISCHE PATENTSCHRIFT

(11) **EP 2 670 382 B1**
(45) Veröffentlichungstag und Bekanntmachung des Hinweises auf die Patenterteilung: **18.07.2018**
(21) Anmeldenummer: 12701517.0
(22) Anmeldetag: 30.01.2012
(51) Int. Cl.: A61K 8/49, A61K 8/63, A61K 8/73, A61Q 19/06, A61Q 19/08

(54) **KOSMETISCHE VERWENDUNG**
COSMETIC USE
UTILISATION COSMÉTIQUE

(30) Priorität: 31.01.2011 DE 102011003408; 21.02.2011 US 201161444795 P
(43) Veröffentlichungstag der Anmeldung: 11.12.2013
(73) Patentinhaber: LUCOLAS-M.D. Ltd., Birmingham B18 6EW (GB)
(72) Erfinder: SCHMIDT, Alfred, F-68740 Nambsheim (FR)
(74) Vertreter: Prüfer & Partner mbB Patentanwälte · Rechtsanwälte
(86) Internationale Anmeldenummer: PCT/EP2012/051421
(87) Internationale Veröffentlichungsnummer: WO 2012/104240

(56) Entgegenhaltungen:
- EP-A1- 0 943 333
- WO-A1-97/36570
- DE-A1-102008 012 988
- DATABASE GNPD [Online] MINTEL; Februar 2009 (2009-02), "Skin Recovery Moisturizer", XP002715528, Database accession no. 1052870

## Beschreibung

Die Erfindung betrifft die kosmetische Verwendung einer Zusammensetzung zur Verbesserung des allgemeinen Zustandes und Erscheinungsbildes der Haut.

Schönheitsideale sind abhängig vom jeweiligen kulturellen Umfeld bzw. der jeweiligen Epoche gewissen Wandlungen unterworfen. Nichtsdestoweniger ist auch heute für die Mehrzahl der Bevölkerung ein makelloses Äußeres von großer Bedeutung. Eine entscheidende Rolle spielen hierbei der Zustand und das Aussehen der Haut.
Bei der Haut handelt es sich um ein sehr vielseitiges Organ, dem im menschlichen bzw. tierischen Organismus eine Reihe essentieller Funktionen zukommen. So stellt die Haut einerseits eine Barriere dar, die den Körper nach außen abgrenzt und vor schädlichen Umwelteinflüssen bewahrt bzw. den Austausch mit der Umwelt ermöglicht. Andererseits übernimmt die Haut wichtige Stoffwechselfunktionen und ist z.B. maßgeblich an der Abwehr von Krankheitserregern aber auch an allergischen Reaktionen beteiligt.
Als Folge von z.B. Nikotin- und/oder Alkohol-Abusus und der permanenten Exposition der Haut gegenüber Umwelteinflüssen wie z.B. UV-Strahlung kommt es zur sogenannten exogenen Hautalterung. Darüber hinaus bewirken endogene Faktoren wie z.B. genetische Prädisposition zusätzlich einen Alterungseffekt.
Eine Folge der Hautalterung ist die Bildung von Falten aufgrund Austrocknung und Elastizitätsverlust in der Oberhaut. Damit gehen verschlechterte Wundheilung und eine insgesamt dünnere Oberhautschicht einher. Dies führt zu einer stärkeren Sichtbarkeit von modifizierten Blutgefäßen, insbesondere im Fall von Besenreisern.
Ein weiterer Zustand, der zu einer signifikanten Beeinträchtigung des Erscheinungsbildes der Haut führt, ist die Cellulite. Bei der Cellulite handelt es sich nicht um einen krankheitsbedingten Zustand, sondern vielmehr um ein ästhetisches Problem, das vor allem bei Frauen auftritt. Dabei kommt es in der Unterhaut zur vermehrten Fetteinlagerung, was bei entsprechender Bindegewebsschwäche zu einer ungleichmäßigen Dellenbildung auf der Haut, der sogenannten Orangenhaut führt.
Auch die sogenannten Dehnungsstreifen stellen für viele Menschen eine Beeinträchtigung ihrer Lebensqualität dar. Dehnungsstreifen entstehen durch die Überdehnung des Bindegewebes in der Unterhaut z.B. infolge starker Gewichtszunahme. Die Überdehnung des Bindegewebes führt zunächst zu blaurötlichen Streifen; durch das Vernarben dieser Geweberisse erscheinen diese später als helle Streifen, die sich je nach Pigmentierung der betroffenen Hautareale unterschiedlich stark von der umgebenden Haut abheben.

Die Kosmetikindustrie bietet verschiedene Möglichkeiten an, mit denen Alterserscheinungen im Allgemeinen entgegen gewirkt werden soll. Der langfristige Erfolg der jeweiligen Produkte und Methoden bleibt jedoch vielfach hinter den Erwartungen der Anwender zurück. So werden z.B. vor allem zwei Methoden zur Entfernung von Besenreisern eingesetzt, die beide mit Nebenwirkungen verbunden sind und bei denen häufige Rezidive auftreten. Bei der Sklerosierung handelt es sich um eine invasive Methode, bei der neben Schmerzen Nebenwirkungen wie Blutergüsse und Venenthrombosen auftreten können. Bei der Laserbehandlung, bei der häufig mehrere Sitzungen erforderlich sind, können als Nebenwirkungen allergische Hautreaktionen und Schmerzen auftreten. Rezidivraten sind bei beiden Methoden relativ häufig.

Es besteht daher ein Bedarf an kosmetischen Verwendungen, die den oben genannten Phänomenen und Alterungserscheinungen der Haut im Allgemeinen entgegenwirken.
Aufgabe der vorliegenden Erfindung ist es, eine kosmetische Verwendung einer Zusammensetzung bereitzustellen, die das Erscheinungsbild der Haut verbessert. Insbesondere sollen die sichtbaren Folgen von Cellulite, Dehnungsstreifen, Besenreisern und Alterserscheinungen im Allgemeinen reduziert oder verhindert werden.
Diese Aufgabe wird durch die vorliegende Erfindung wie in Anspruch 1 definiert gelöst. Weiterbildungen sind in den entsprechenden Unteransprüchen definiert.

Die vorliegende Erfindung betrifft die Verwendung gemäß Anspruch 1. Bei der erfindungsgemäßen Verwendung können die einzelnen Komponenten jeweils unabhängig voneinander gemeinsam, d.h. in einer gemeinsamen Zusammensetzung, oder alternativ separat in getrennten Formen, allerdings dann so verwendet werden, dass sie mindestens in einem gemeinsamen Zeitraum zur Anwendung kommen. Die Verwendung in einer gemeinsamen Zusammensetzung ist bevorzugt.
Der Zustand der Haut wird wesentlich durch die Mikrozirkulation in der Haut bestimmt. Diese wird z.B. durch die Vergrößerung bzw. Vermehrung der Fettzellen obstruiert. Die Zunahme der Fettzellen bzw. des Fettgehalts in den Zellen reduziert das Wasserbindungsvermögen der Haut und führt zu Faltenbildung.
Im Rahmen der vorliegenden Erfindung wurde überraschend gefunden, dass die Verwendung der erfindungsgemäßen Zusammensetzung, die neben einem Aromatase-Inhibitor und einem 5-α-Reduktase-Inhibitor auch ein Antioxidans und Hyaluronsäure enthält, das allgemeine Erscheinungsbild der Haut deutlich verbessert. Die erfindungsgemäße Verwendung führt nicht nur zur Faltenreduktion, sondern bewirkt auch eine Verbesserung von Cellulite, Dehnungsstreifen, Besenreisern sowie des allgemeinen Erscheinungsbildes der Hautoberfläche. Insbesondere Alterserscheinungen (z.B. Altersflecken, Augenfältchen, allg. Gesichtsfalten etc.), Dekollete Alterung oder UV-Alterungserscheinungen (Foto-Aging) werden durch die erfindungsgemäße Verwendung verbessert.
Ohne den Umfang der vorliegenden Erfindung durch eine Theorie beschränken zu wollen, wird angenommen, dass der erfindungsgemäße Effekt auf einer Verbesserung der Mikrozirkulation in der Haut beruht. Diese führt ihrerseits nicht nur zu erhöhter Wasserbindung in der Haut, sondern auch zu einer Stärkung der Kollagenfasern und somit zu einer glatten Hautoberfläche. Darüber hinaus bewirkt die durch die erfindungsgemäße Verwendung verbesserte Mikrozirkulation offensichtlich auch eine Förderung von Stoffwechselprozessen in der Haut. Es wird angenommen, dass schädliche Stoffwechselprodukte, die bei mangelhafter Mikrozirkulation in der Haut akkumulieren, verstoffwechselt und/oder über die Lymphe abtransportiert werden.
Dadurch wird zusätzlich zur hormonellen Balance auch das metabolische Gleichgewicht der Haut wiederhergestellt und das Bindegewebe gestärkt. Es wird vermutet, dass gleichzeitig durch die Wirkung des Antioxidans die Wirkung von schädlichen Radikalen reduziert oder gänzlich verhindert wird, was zum Schutz der Haut vor neuen Schädigungen dient. Durch diesen Ansatz, der auf die Beseitigung zentraler Ursachen der meisten Hautschädigungen abzielt, können somit verschiedenste Phänomene behandelt werden, die das Erscheinungsbild der Haut beeinträchtigen. Durch eine verbesserte Versorgung der Haut und einen verbesserten Schutz der Haut vor Schädigungen durch Radikale und vor Feuchtigkeitsentzug wird der Allgemeinzustand der Haut verbessert.
Es wird darüber hinaus vermutet, dass die unerwartete Verbesserung des Erscheinungsbildes der Haut durch ein synergistisches Zusammenwirken der einzelnen Komponenten der erfindungsgemäßen Zusammensetzung bewirkt wird. Durch die Wirkung des Aromatase-Inhibitors bzw. des 5-α-Reduktase-Inhibitors wird die Umwandlung von Testosteron in Östrogen lokal reduziert oder verhindert. Dies führt u.a. zu verminderter Zunahme der Fettzellen bzw. zu einer Abnahme des Volumens/Fettgehalts der Fettzellen. Gleichzeitig werden anabole Vorgänge gefördert, die zur Straffung des Bindegewebes und zur Wundheilung beitragen. Insbesondere die gleichzeitige Verwendung der Hyaluronsäure ist wichtig, steigert sie doch die Effekte des Aromatase-Inhibitors bzw. des 5-α-Reduktase-Inhibitors zudem dadurch, dass sie die Resorption dieser Wirkstoffe und die des Antioxidans verbessert und die Wirkstoffe in der Haut "festhält".
Das Antioxidans schützt wie oben erwähnt vor freien Radikalen und reduziert oder verhindert so eine erneute Schädigung der Haut.
Die Wirkungen der einzelnen Wirksubstanzen verstärken sich dabei offenbar überproportional, was zu der unerwarteten Verbesserung des Allgemeinzustands der Haut führt.

Die Erfindung soll anhand der nachfolgenden Beschreibung von bevorzugten Ausführungsformen im Detail veranschaulicht werden, ohne jedoch das allgemeine erfindungsgemäße Konzept darauf zu beschränken.

Die vorliegende Erfindung betrifft die Verwendung gemäß Anspruch 1 eines Mittels bzw. einer Zusammensetzung, das bzw. die neben einem Aromatase-Inhibitor und einem 5-α-Reduktase-Inhibitor auch ein Antioxidans und Hyaluronsäure enthält, zu kosmetischen Zwecken.
Im Sinne der vorliegenden Erfindung wirkt die Verwendung eines Antioxidans in Kombination mit einem Aromatase-Inhibitor und eines 5-α-Reduktase-Inhibitors und der weiteren Komponente, der Hyaluronsäure, dem Alterungsprozeß der Haut entgegen und verbessert - vor allem wegen der Kombination mit der Hyaluronsäure - die Mikrozirkulation in der Haut.
Aromatase-Inhibitoren im Sinne der vorliegenden Erfindung sind steroidale Aromatase-Inhibitoren (Aromatase-Inaktivatoren) wie z.B 4-Hydroxyandrostendion, Exemestan, 4-Acetoxyandrostendion, 5-α-Androst-3-en-17-on und 3-α,4-α-Epoxy-5-α-androstan-17-on.
5-α-Reduktase-Inhibitoren im Sinne der vorliegenden Erfindung, die erfindungsgemäß verwendet werden können, sind der Extrakt von Früchten der Sägepalme (*Serenoa repens,* syn. *Sabal serrulata*), Wurzeln der Brennnessel (Urtica dioica), Rindenextrakt der afrikanischen Pflaume (Pygeum africanium) und Kürbiskernextrakt (Cucurbita pepo seed). Gemäß der vorliegenden Erfindung ist der steroidale Aromatase-Inaktivator vorzugsweise von einer Wirkstärke, die durch die mittlere inhibitorische Konzentration IC(50) im Bereich von 0,2 nM bis 500 nM gekennzeichnet ist.
Der 5-α-Reduktase-Inhibitor im Sinne der Erfindung ist gekennzeichnet durch eine mittlere inhibitorische Konzentration IC(50) im Bereich von 5 nM bis 500 nM.
Ein Antioxidans gemäß der vorliegenden Erfindung ist ein "Radikalfänger", der freie Radikale einfängt oder deren schädlichen Einfluss in der Zelle beendet. Die Antioxidans-Substanz sollte vom Aromatase-Inhibitor verschieden sein. Während die Wirkungsweise der Antioxidantien wie erläutert im Sinne der Erfindung gleichgerichtet und konsistent ist, ist die Gruppe der Antioxidantien strukturell sehr heterogen. Geeignete Substanzen im Sinne der Erfindung werden anhand ihrer Fähigkeit, die Oxidation anderer Moleküle zu verhindern, ausgewählt. Der Fachmann ist in der Lage, ein Antioxidans mittels etablierter und publizierter Methoden zu identifizieren. Die Verfahren sind dem Fachmann bekannt und sollen hier nicht im Detail erläutert werden. So kann z.B. die Menge an freien Radikalen durch EPR (*electric paramagnetic resonance*) gemessen werden (Lo Scalzo, EJEAFChe 2010; 9:1360-1371). Dazu werden Substanzen wie z.B. 5,5-Dimethyl-1-pyrrolin-N-oxid (DMPO) oder 1,1-Diphenyl-2-picrylhydrazyl (DPPH) eingesetzt, die eine hohe Affinität zu freien Radikalen haben und mit diesen stabile Verbindungen eingehen, die spektrometrisch messbar sind. Es kommen auch Verfahren zum Einsatz, bei denen die zu messende Substanz chromatographisch (z.B. durch HPLC) gereinigt wird (Yamaguchi et al., Bioscience, Biotechnology, and Biochemistry 1998; 62:1201-1204). Im Sinne der Erfindung werden antioxidative Substanzen verwendet. Bei den verwendeten Antioxidantien kann es sich um Substanzen unterschiedlicher chemischer Klassen und unterschiedlichen Ursprungs handeln. Dies können auch antioxidative Substanzen sein, die im Organismus vorkommen, deren Menge bzw. Verfügbarkeit jedoch durch die zusätzliche Verabreichung im Rahmen der erfindungsgemäßen Verwendung wirksam erhöht werden, und/oder gegebenenfalls gerade am gewünschten Zielort durch geeignete Applikation bereitgestellt werden. Nicht-enzymatische Antioxidantien umfassen insbesondere Flavonoide (z.B. oligomere Proanthocyanidine (OPC), Anthocyane oder Polyphenole wie Quercetin oder Catechin); Vitamine (z.B. Vitamin C, Vitamin E); Carotenoide (z.B. b-Carotin, Lycopen, Lutein); Minerale (z.B. Kupfer, Mangan, Zink, Selen); Hormone (z.B. Melatonin); Steroide (z.B. Kortison); Ubichinone; N-Acetylcystein; α-Liponsäure; ein Grüntee-Extrakt, der eine antioxidativ wirksame Zusammensetzung aus Polyphenolen, optional auch Aminosäuren, Mineralstoffe (Spurenelemente) und Polysaccharide, enthält und vor allem der die speziellen hochantioxidativ wirkenden Polyphenole Epicatechin und Epigallocatechin enthält (z.B. OM24®, erhältlich bei Omnimedica, Schweiz); und Glutathion. Einige Enzyme erfüllen die Funktion von Antioxidantien und werden als enzymatische Antioxidantien bezeichnet, wie z.B. Glutathionperoxidase, Superoxiddismutase und Katalase. Im Vergleich zu möglicherweise nur endogen vorkommenden, oder gegebenenfalls nur zufällig oder für andere Zwecke zugegebenen Antioxidantien kann erfindungsgemäß durch Einstellung passender Mengen oder durch topische Anwendung sichergestellt werden, dass ein gewünschter kosmetischer Effekt erzielt wird.

In einer besonderen Ausführungsform ist das Antioxidans, das in der kosmetischen Zusammensetzung verwendet wird, α-Liponsäure (1,2-Dithiolan-3-pentansäure) oder Grüntee-Extrakt enthaltend Polyphenole, insbesondere OM24®. α-Liponsäure ist sowohl in den wässrigen als auch in den Fettphasen der Zellen aktiv. Die Substanz wird über die Haut hervorragend resorbiert. Dies trifft vor allem in Kombination mit der Hyaluronsäure zu. Dies eröffnet unterschiedliche Möglichkeiten der Verabreichung. α-Liponsäure wird im Organismus rasch in Dihydroliponsäure umgewandelt. Dihydroliponsäure regeneriert andere, weitere Antioxidantien wie Vitamin C und Vitamin E, was zu weiteren verstärkten Effekten bei der Verabreichung von α-Liponsäure führen kann. α-Liponsäure induziert darüber hinaus die Bildung von Glutathion im Gewebe. Zudem regeneriert α-Liponsäure Glutathion aus Glutathiondisulfid.

Bei der topischen Verwendung der Zusammensetzung auf der Haut werden die Wirkstoffe auf das zu behandelnde Hautareal aufgetragen. Die Wirkstoffmenge wird dabei vorzugsweise so gewählt, dass keine Plasmaspiegel, sondern nur lokal wirksame Konzentrationen erreicht werden. Dieses bewirkt, dass unerwünschte systemische Wirkungen vermieden werden. Auch bei der Verwendung über einen längeren Zeitraum werden so unerwünschte Nebenwirkungen verhindert.

Alternativ können andere Formen der Applikation zum Einsatz kommen wie z.B. das Aufsprühen der Wirkstoffe auf die zu behandelnden Hautflächen.

Die Wirkstoffe können dabei voneinander getrennt oder in einer gemeinsamen Zusammensetzung aufgetragen werden, solange sichergestellt ist, dass die aktiven Wirkstoffe das Zielgewebe gleichzeitig erreichen bzw. die Zeiträume, in denen die einzelnen Wirkstoffe im Zielgewebe als aktive Substanzen vorliegen, zumindest überlappen.

In einer bevorzugten Ausführungsform umfasst die erfindungsgemäße Verwendung α-Liponsäure und Hyaluronsäure in Kombination mit einem Aromatase-Inhibitor und/oder einem 5-α-Reduktase-Inhibitor. In einer besonders bevorzugten Ausführungsform umfasst die erfindungsgemäße Verwendung α-Liponsäure, Hyaluronsäure und 4-Acetoxyandrostendion. Die Verwendung von Grüntee-Extrakt enthaltend Polyphenole, insbesondere OM24® als Antioxidans anstelle von, oder zusätzlich zu der Verwendung von α-Liponsäure ist ebenfalls bevorzugt.

Weitere andere Hilfsstoffe, die üblicherweise bei kosmetischen Zusammensetzungen (Cremes, Salben, Gele, Schäume, Tinkturen, Lotionen usw.)verwendet werden, können mit den oben genannten Wirkstoffen kombiniert werden. Insbesondere kann die erfindungsgemäße Zusammensetzung Hilfsmittel umfassen, die bei topischen Applikationsformen üblicherweise zum Einsatz kommen. Ebenso können für die Herrichtung als Spray entsprechende Träger und Hilfsmittel verwendet werden, die dem Fachmann bekannt sind.

Im Sinne der Erfindung liegen die bevorzugten Konzentrationen des steroidalen Aromatase-Inaktivators in der Zusammensetzung in einem Bereich von 0,25 Gew.-% bis 1,5 Gew.-% (z.B. bei Cellulite 0,6 Gew.-%).
Die Konzentration des 5-α-Reduktase-Inhibitors liegt erfindungsgemäß im Bereich von 0,5 Gew.-% bis 5 Gew.-%.
Das Antioxidans liegt bevorzugterweise in einer Konzentration von von 0,2 Gew.-% bis 2,5 Gew.-% in der Zusammensetzung vor. Die Zusammensetzung wird typischerweise 1-2-mal täglich auf die entsprechenden Hautareale aufgebracht. Dabei werden in der Regel 1-5 g z.B. einer Creme bzw. 2-5 ml z.B. eines Sprays je Anwendung verwendet.
Die vorliegende Erfindung wird durch die nachfolgenden Beispiele weiter veranschaulicht, ohne durch diese beschränkt zu werden.

Die folgenden illustrativen Beispiele 1 und 2 veranschaulichen die Wirksamkeit der erfindungsgemäßen Verwendung im Falle von Besenreisern. Folgende Zusammensetzung wurde dabei verwendet:
1,0 % Acetoxyandrostendion
0,5 % α-Liponsäure
0,5 % Hyaluronsäure
als Wirkstoffe
in DAC-Basiscreme:
   4,0 g Glycerolmonostearat
   6,0 g Cetylalkohol
   7,5 g mittelkettige Triglyceride (Neutralöl, Miglyol)
   25,5 g weisse Vaseline
   7,0 g Macrogol-20-glycerolmonostearat
   10,0 g Propylenglycol
   40,0 g gereinigtes Wasser.

### Beispiel 1:

S.H.C., 48 Jahre, weiblich: Besenreiser im Innenbereich der Schenkel in Höhe des Kniegelenks beidseits sowie eine starke kreisförmige Anhäufung (Durchmesser ca. 1,5 cm) auf der Rückseite des linken Oberschenkels knapp oberhalb des Kniegelenks.
Nach vierwöchiger Behandlung mit der erfindungsgemäßen Creme 2mal täglich: an der Rückseite hat sich die kreisförmige Anhäufung bereits aufgelöst, es sind nur noch Besenreiser sichtbar, die langgezogen sind, aber immer heller werden.
Die Behandlung wird fortgesetzt, da völlig ohne Nebenwirkungen.

### Beispiel 2:

E.C., 51 Jahre, weiblich: Besenreiser an den Außenseiten beider Oberschenkel und an den Waden.
Nach Behandlung mit der erfindungsgemäßen Creme über einen Zeitraum von 3 Monaten, 2mal täglich: fast keine sichtbaren Besenreiser mehr.
Die Behandlung wird mit einmal täglicher Gabe fortgesetzt, da völlig ohne Nebenwirkungen.

Das folgende illustrative Beispiel 3 zeigt die Ergebnisse bei Probandinnen mit Cellulite. Folgende Creme-Zusammensetzung wurde dabei 1-mal täglich verwendet (ca. 1,5 g pro betroffener Körperseite; %-Angaben in Gew.-%):
0,6 % Acetoxyandrostendion
0,5 % α-Liponsäure
0,2 % Hyaluronsäure
als Wirkstoffe
in DAC-Basiscreme:
4,0 g Glycerolmonostearat
6,0 g Cetylalkohol
7,5 g mittelkettige Triglyceride (Neutralöl, Miglyol)
25,5 g weisse Vaseline
7,0 g Macrogol-20-glycerolmonostearat
10,0 g Propylenglycol
40,0 g gereinigtes Wasser.

### Beispiel 3: Beobachtungsstudie an 50 Probandinnen.

Die Probandinnen wurden in 5 Schweizer Gesundheits-Studios behandelt. Aufgenommen wurden nur Probandinnen mit einer Cellulite Ausprägung der Scorepunkte 2 und 3 der Cellulite Beurteilungsscores nach Nürnberger und Müller (Nürnberger F., Müller G.: So-called Cellulite: an invented disease. J.Dermatol. Surg. Oncol. 1978, 4: 221-9).
Nürnberger Score: 0 = keine Orangenhaut (Cellulite)
1 = geringe Ausprägung
2 = mäßige Ausprägung
3 = starke Ausprägung
Der Studienverlauf betrug 12 Wochen. Beobachtungszeitpunkte 0: vor Behandlung / Behandlungsbeginn; weitere Untersuchungszeitpunkte nach Woche 4, 8 und 12.
Die Probandinnen hatten ein Durchschnittsalter von 36 Jahren (19-57 Jahre), waren gesund und normal bis leicht übergewichtig.
Therapieergebnisse (nach 12 Wochen; beurteilt nach Nürnberger Score):
Zu Therapiebeginn: 29 Probandinnen; Score 2
21 Probandinnen; Score 3
Alle Probandinnen durchliefen die 12 wöchige Beobachtungsperiode; Ergebnisse nach 12 Wochen Anwendung: Von den 29 Probandinnen mit mäßiger Ausprägung zu Beginn hatten 20 Probandinnen keine Cellulite mehr (Score 0), 9 Probandinnen Score 1, geringe Ausprägung.
Von den 21 Probandinnen Score 3 hatten nach 12 Wochen 8 Probandinnen Score 2; 11 Score 1; 2 Score 0.

Über unerwünschte Wirkungen klagte keine der Probandinnen; die Creme wurde als sehr angenehm und wirksam empfunden.
Zur Erhaltung bzw. weiteren Verbesserung der Resultate führen die Probandinnen die Anwendung fort und zwar mit der gleichen Creme-Zusammensetzung.

## Patentansprüche

1. Verwendung einer Zusammensetzung, welche die folgenden Wirkstoffe umfasst:
- einen steroidalen Aromatase-Inaktivator und einen 5-α-Reduktase-Inhibitor,
- ein Antioxidans und
- Hyaluronsäure
für kosmetische Zwecke, wobei die Zusammensetzung topisch auf die Haut verabreicht wird, das Antioxidans in einer Menge von 0,2 Gew.-% bis 2,5 Gew.-% in der Zusammensetzung vorliegt, der 5-α-Reduktase-Inhibitor ausgewählt ist aus der Gruppe bestehend aus dem Extrakt von Früchten der Sägepalme (Serona repens, syn. Sabal serrulata), Wurzeln der Brennnessel (Urtica dioica), Rindenextrakt der afrikanischen Pflaume (Pygeum africanium), und Kürbiskernextrakt (Cucurbita pepo seed), wobei der 5-α-Reduktase-Inhibitor **dadurch gekennzeichnet ist, dass** die mittlere inhibitorische Konzentration IC(50) im Bereich von 5 nM bis 500 nM liegt, und wobei die Zusammensetzung für kosmetische Zwecke bei Cellulite, Dehnungsstreifen, Alterungserscheinungen der Haut, Falten, Besenreisern, und dem allgemeinen Erscheinungsbild der Hautoberfläche verwendet wird.

2. Verwendung gemäß Anspruch 1, wobei der steroidale Aromatase-Inaktivator ausgewählt ist aus der Gruppe bestehend aus 4-Hydroxyandrostenedion, Exemestan, 4-Acetoxyandrostendion, 5-α-Androst-3-en-17-on und 3-α,4-α-Epoxy-5-α-androstan-17-on.

3. Verwendung gemäß Anspruch 1 oder 2, wobei der steroidale Aromatase-Inaktivator **dadurch gekennzeichnet ist, dass** die mittlere inhibitorische Konzentration IC(50) im Bereich von 0,2 nM bis 500 nM liegt.

4. Verwendung gemäß irgendeinem der vorangehenden Ansprüche, wobei das Antioxidans ausgewählt ist aus der Gruppe bestehend aus Flavonoiden; Carotenoiden; Steroiden; α-Liponsäure; und Grüntee-Extrakt enthaltend Polyphenole.

5. Verwendung gemäß irgendeinem der vorangehenden Ansprüche, wobei der 5-α-Reduktase-Inhibitor die Aktivität von 5-α-Reduktase Typ I und/oder Typ II hemmt.

6. Verwendung gemäß irgendeinem der vorangehenden Ansprüche, wobei die Zusammensetzung
4-Acetoxyandrostendion,
Sägepalmenextrakt,
α-Liponsäure und/oder Grüntee-Extrakt enthaltend Polyphenole, und Hyaluronsäure umfasst.

## Claims

1. Use of a composition comprising the following active ingredients:
- a steroidal aromatase-inactivator and a 5-α-reductase-inhibitor,
- an antioxidant, and
- hyaluronic acid
for cosmetic purposes, wherein the composition is applied topically to the skin, the antioxidant is present in the composition in an amount of 0.2 wt.-% to 2.5 wt.-%, the 5-α-reductase-inhibitor is selected from the group consisting of the extract from the fruits of saw palmetto (Serona repens, syn. Sabal serrulata), roots of stinging nettle (Urtica dioica), bark extract of African plum (Pygeum africanium), and extract from pumpkin seed (Cucurbita pepo seed), wherein the 5-α-reductase-inhibitor is **characterized in that** the mean inhibitory concentration IC(50) is in the range from 5 nM to 500 nM, and wherein the composition is for cosmetic purposes in cellulite, stretch marks, signs of skin aging, wrinkles, spider veins, and the general appearance of the skin surface.

2. Use according to claim 1, wherein the steroidal aromatase-inactivator is selected from the group consisting of 4-hydroxyandrostenedione, exemestane, 4-acetoxyandrostenedione, 5-α-androst-3-ene-17-one and 3-α,4-α-epoxy-5-α-androstane-17-one.

3. Use according to claim 1 or 2, wherein the steroidal aromatase-inactivator is **characterized in that** the mean inhibitory concentration IC(50) is in the range from 0.2 nM to 500 nM.

4. Use according to any of the preceding claims, wherein the antioxidant is selected from the group consisting of flavonoids; carotenoids; steroids; α-lipoic acid; and green tea extract containing polyphenols.

5. Use according to any of the preceding claims, wherein the 5-α-reductase-inhibitor inhibits the activity of 5-α-reductase type I and/or type II.

6. Use according to any of the preceding claims, wherein the composition comprises
4-acetoxyandrostenedione,
saw palmetto extract,
α-lipoic acid and/or green tea extract containing polyphenols,
and hyaluronic acid.

## Revendications

1. Utilisation d'une composition qui comprend les principes actifs suivants :
- un inactivateur d'aromatase stéroïdienne et un inhibiteur de 5-α-réductase,
- un antioxydant et
- de l'acide hyaluronique
à des fins cosmétiques, dans laquelle la composition est appliquée en mode topique sur la peau, l'antioxydant est présent dans la composition en quantité de 0,2 % en poids à 2,5 % en poids, l'inhibiteur de 5-α-réductase est choisi dans le groupe constitué de l'extrait de fruit du chou palmiste (Serona repens, syn. Sabal cerrulata), des racines d'ortie urticante (Urtica dioica), de l'extrait d'écorce du prunier africain (Pygeum africanium) et de l'extrait de graine de potiron (semence de Cucurbita pepo), dans lequel l'inhibiteur de 5-α-réductase est **caractérisé en ce que** la concentration inhibitrice moyenne IC(50) se situe dans la plage de 5 nm à 500 nm et dans laquelle la composition est utilisée à des fins cosmétiques dans le cadre de la cellulite, des vergetures, des phénomènes de vieillissement de la peau, des rides, des télangiectasies et des symptômes généraux de la surface de la peau.

2. Utilisation selon la revendication 1, dans laquelle l'inactivateur d'aromatase stéroïdienne est choisi dans le groupe constitué de la 4-hydroxyandrostènedione, de l'exemestane, de la 4-acétoxyandrostènedione, de la 5-a -androst-3-én-17-one et de la 3-α,4-α-époxy-5-α-androstan-17-one.

3. Utilisation selon la revendication 1 ou 2, dans laquelle l'inactivateur d'aromatase stéroïdienne est **caractérisé en ce que** la concentration inhibitrice moyenne IC(50) se situe dans la plage de 0,2 nm à 500 nm.

4. Utilisation selon l'une quelconque des revendications précédentes, dans laquelle l'antioxydant est choisi dans le groupe constitué des flavonoïdes ; des caroténoïdes ; des stéroïdiens ; de l'acide α-lipoïque et d'un extrait de thé vert contenant des polyphénols.

5. Utilisation selon l'une quelconque des revendications précédentes, dans laquelle l'inhibiteur de 5-α-réductase bloque l'activité de la 5-α-réductase de type I et/ou de type II.

6. Utilisation selon l'une quelconque des revendications précédentes, dans laquelle la composition comprend :
de la 4-acétoxyandrostènedione,
un extrait de chou palmiste,
de l'acide α-lipoïque et/ou un extrait de thé vert contenant des polyphénols, ainsi que de l'acide hyaluronique.
